# EUROPEAN PATENT APPLICATION

(11) **EP 3 729 995 A1**
(43) Date of publication of application: **28.10.2020**
(21) Application number: 18890418.9
(22) Date of filing: 26.11.2018
(51) Int. Cl.: A45D 44/00, A61F 13/02, A61K 8/02, A61K 8/19, A61K 8/34, A61K 8/55, A61K 8/81, A61Q 1/02, C09D 11/033

(54) **COSMETIC SHEET AND METHOD OF MANUFACTURING SAME, COSMETIC MATERIAL INK, INKJET PRINTING INK, AND COSMETIC SHEET MANUFACTURING DEVICE**

(30) Priority: 21.12.2017 JP 2017245466
(71) Applicant: Panasonic Intellectual Property Management Co., Ltd., Osaka-shi, Osaka 540-6207 (JP)
(72) Inventor: ONODERA, Mari, Osaka-shi, Osaka 540-6207 (JP); SHINODA Masayo, Osaka-shi, Osaka 540-6207 (JP)
(74) Representative: Grünecker Patent- und Rechtsanwälte PartG mbB
(86) International application number: PCT/JP2018/043360
(87) International publication number: WO 2019/123984

(57) **Abstract**

There are provided a cosmetic sheet that exhibits luster and transparency and has good appearance when attached to the skin and a method for producing the cosmetic sheet. There are also provided a cosmetic ink and an ink jet printing ink for producing the cosmetic sheet. A cosmetic sheet (50) to be attached to a skin (510) includes a thin film (520), one surface of which is to be attached to a skin, a light-scattering layer (522) disposed on the thin film and containing a reflective material, a coloring layer (523) disposed on the light-scattering layer and containing a colorant, and a glossy layer (524) disposed on the coloring layer and containing a gloss agent.

## Description

### Technical Field

The present disclosure relates to a cosmetic sheet and a method for producing the cosmetic sheet, a cosmetic ink, an ink jet printing ink, and an apparatus for producing a cosmetic sheet.

### Background Art

It has been proposed that inks containing various colorants are applied to a thin film, and the resulting thin film is attached to the human body to obscure, for example, a blotch, macula, or scar (hereinafter, also referred to as a "discolored region") on the skin (for example, Patent Literature 1). In the technique disclosed in Patent Literature 1, an image of the skin is taken to identify a discolored region. A color similar to the color of the periphery of the discolored region is printed on a thin film, and the thin film is attached to the skin to obscure the discolored region.

### Citation List

### Patent Literature

PTL 1: Japanese Unexamined Patent Application Publication No. 2015-43836

### Summary of Invention

In the technique disclosed in Patent Literature 1, a coating film having a deep color tone is formed on a thin film to conceal the color of a discolored region. However, a cosmetic sheet produced in which manner tends to cause a feeling of thickness, resulting in a problem that the region to which the cosmetic sheet is attached tends to be conspicuous.

In the attachment of a cosmetic sheet, a cosmetic sheet having luster and transparency enhances the appearance of the region to which the sheet is attached and makes the difference from a region to which no cosmetic sheet is attached obscure. In contrast to this, in the technique disclosed in Patent Literature 1 in which a coating film of a predetermined color is merely formed, it is difficult to provide a cosmetic sheet having luster and transparency.

An aspect of the present disclosure provides a cosmetic sheet that exhibits luster and transparency and has good appearance when attached and provides thereof. Another aspect of the present disclosure provides a cosmetic ink for producing the sheet and an ink jet printing ink including the cosmetic ink.

A cosmetic sheet according to an aspect of the present disclosure is a cosmetic sheet to be attached to a skin, the cosmetic sheet including a thin film, one surface of which is to be attached to a skin, a light-scattering layer disposed on the thin film and containing a reflective material, a coloring layer disposed on the light-scattering layer and containing a colorant, and a glossy layer disposed on the coloring layer and containing a gloss agent.

According to an aspect of the present disclosure, there is provided a cosmetic sheet that exhibits luster and transparency and has good appearance when attached.

### Brief Description of Drawings

[Fig. 1A] Fig. 1A is a view illustrating the reflection of light when no cosmetic sheet is attached.
[Fig. 1B] Fig. 1B is a view illustrating the reflection of light when a cosmetic sheet is attached.
[Fig. 2] Fig. 2 is a schematic sectional view illustrating a cosmetic sheet according to an embodiment of the present disclosure.

### Description of Embodiments

### 1. Cosmetic sheet

A cosmetic sheet according to the present disclosure is a sheet to be attached to the skin for the purpose of coloration and beautification of the skin. The cosmetic sheet may be a sheet for use by a particular individual (a sheet produced on demand), the sheet being produced according to a discolored portion of the skin of the particular individual by using a publicly known makeup support system or the like. On the other hand, the cosmetic sheet may be, for example, a sheet for use by large numbers of the general public, the sheet being produced according to an ordinary skin color of large numbers of the general public. The cosmetic sheet can be used not only for, for example, coloration of the skin but also for applications to obscure a discolored portion or to decorate a discolored portion when the cosmetic sheet is attached on the discolored portion of the skin.

As described above, a cosmetic sheet including a thin film and a coloring layer disposed on the thin film is known. In the cosmetic sheet, the color tone of the coloring layer is deepened to thereby conceal the original color of the skin and to adjust the hue and color tone of a portion to which the cosmetic sheet is attached to desired ranges. However, such a coloring layer that merely has a deepened color tends to cause a feeling of thickness and tends to be conspicuous when the cosmetic sheet is attached. In addition, such a cosmetic sheet has poor luster and transparency, and it is desirable to provide a cosmetic sheet having a better appearance.

In view of this, the cosmetic sheet according to the present disclosure includes a thin film and a light-scattering layer, a coloring layer, and a glossy layer that are stacked on the thin film in this order. With this layer structure, the hue and color tone of a portion to which the cosmetic sheet is attached can be adjusted to desired ranges without causing a feeling of thickness. In particular, the cosmetic sheet has good luster and transparency and capable of significantly enhancing the appearance of a portion to which the cosmetic sheet is attached. The reason for this will be specifically described.

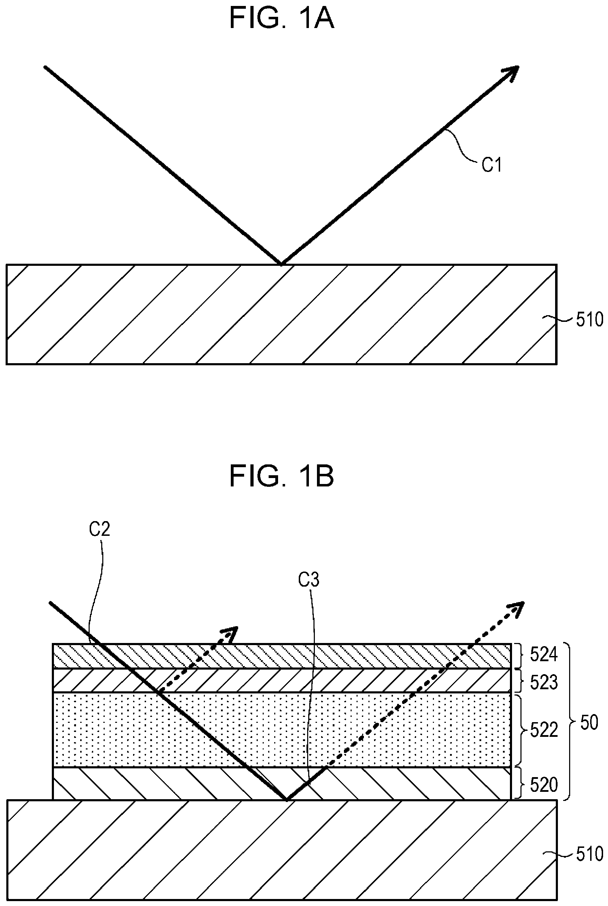

Fig. 1A shows a view illustrating the reflection of light when no cosmetic sheet is attached. Fig. 1B shows a view illustrating the reflection of light when a cosmetic sheet according to the present disclosure is attached. As illustrated in Fig. 1A, for example, when a cosmetic sheet 50 is not attached to a skin 510, light C1 reflected at a surface of the skin 510 is directly visually recognized.

In contrast, when a cosmetic sheet 50 in which a thin film 520, a light-scattering layer 522, a coloring layer 523, and a glossy layer 524 are stacked in this order is attached to a skin 510, most of light C2 incident on the cosmetic sheet 50 is reflected at the surface of the light-scattering layer 522 (refer to Fig. 1B). Furthermore, light C3 passing through the light-scattering layer 522 and reflected at the surface of the skin 510 is reflected or scattered by the light-scattering layer 522 while the light C3 travels toward the surface of the cosmetic sheet 50. That is, the incident light C2 is unlikely to reach the surface of the skin 510. Even if the light C2 reaches the surface of the skin 510, most of the reflected light C3 does not reach the surface of the cosmetic sheet 50. Accordingly, color unevenness, discoloration, and the like of the skin 510 are unlikely to be visually recognized from the surface of the cosmetic sheet 50. In addition, color unevenness and turbidity are small on the surface of the light-scattering layer 522. Accordingly, the coloring layer 523 disposed on this light-scattering layer 522 develops a very good color. Thus, the desired hue and color tone can be reproduced without deepening the color tone of the coloring layer 523.

Furthermore, the cosmetic sheet 50 according to the present disclosure further includes the glossy layer 524 disposed closer to the surface than the coloring layer 523. Therefore, light incident on the surface of the glossy layer 524 and light traveling from the coloring layer 523 toward the surface of the cosmetic sheet 50 are moderately scattered to enhance glossiness in a region to which the cosmetic sheet 50 is attached and to exhibit luster and transparency. As a result, the texture of the region to which the cosmetic sheet 50 is attached can be made close to the texture of the real skin 510, and furthermore, the appearance can be enhanced.

The cosmetic sheet may include a layer other than the thin film, the light-scattering layer, the coloring layer, and the glossy layer. For example, a cosmetic sheet to be attached to a discolored portion of the skin may include a complementary color layer that contains a colorant complementary to the color of the discolored portion of the skin and that is disposed between the thin film and the light-scattering layer. Each layer of the cosmetic sheet according to the present disclosure will now be described in detail.

### 1. Thin Film

The thin film 520 is preferably a biocompatible sheet-like member that does not cause an uncomfortable feeling when attached to the skin of humans. The thin film 520 may be colored within a range that does not impair the objects and advantages of the present disclosure. The thin film 520 may be clear and colorless, or translucent.

The thin film 520 preferably has a thickness of 10 nm to 10 µm, more preferably 10 nm to 1,000 nm. In particular, if the thin film 520 has a hydrophobic property, the thickness is particularly preferably 10 nm to 800 nm. The shape of the thin film 520 in plan view is not particularly limited and may be, for example, a rectangular shape. The thin film 520 may have a shape conforming to the shape of a portion to which the cosmetic sheet 50 is attached or conforming to a pattern of the coloring layer 523. Furthermore, the thin film 520 may have a cut in an outer peripheral portion and/or in a plane thereof so as to conform to the shape of a portion to which the cosmetic sheet 50 is attached.

The thin film 520 may be, for example, a sheet formed by a spin coating method, a roll-to-roll method, a Langmuir-Blodgett (LB) method, or the like or may be a fiber sheet formed of folded fibers produced by an electrospinning method or the like.

Examples of the material of the thin film 520 include polyesters such as polyglycolic acid, polylactic acid, polycaprolactone, polyethylene succinate, polyethylene terephthalate, and copolymers thereof; polyethers such as polyethylene glycol and polypropylene glycol; polyamides such as nylons, polyglutamic acid, polyaspartic acid, and salts thereof; polysaccharides such as pullulan, cellulose, starch, chitin, chitosan, alginic acid, hyaluronic acid, cornstarch, and salts thereof; silicones such as acrylic silicone and trimethylsiloxysilicic acid; acrylic acids such as alkyl acrylates, silicone acrylate, acrylamide, and copolymers thereof; polyvinyl alcohol; polyurethanes; polycarbonates; polyacid anhydrides; polyethylene; polypropylene; porous layer coating sheets, and nanofiber sheets. The material of the thin film 520 is preferably polylactic acid, cellulose (for example, carboxymethylcellulose or hydroxyethylcellulose), starch, chitin, chitosan, alginic acid, cornstarch, or a polyurethane in view of, for example, biocompatibility, availability, and handleability.

### 2. Light-Scattering Layer

The light-scattering layer 522 is a layer disposed on the thin film 520 and containing at least a reflective material. The light-scattering layer 522 may be disposed adjacent to the thin film 520 or may be disposed on the thin film 520 with, for example, a complementary color layer 521 described below therebetween.

The light-scattering layer 522 may be, for example, a layer containing a reflective material and a binder. The light-scattering layer 522 may further contain, for example, a film-forming agent, a dispersant, and various additives. The light-scattering layer 522 may be formed of one layer or two or more layers. When the light-scattering layer 522 is formed of a plurality of layers, the reflective materials contained in the layers may be of the same type or of different types. The amounts of reflective materials contained in the layers may be the same or different.

The reflective material contained in the light-scattering layer 522 may be formed of particles that scatter or reflect ultraviolet light and visible light (for example, light with a wavelength in the range of 200 nm to 780 nm) and may be, for example, a pearl agent, a soft focus agent, or a lame agent. Preferably, the pearl agent, the soft focus agent, and the lame agent do not irritate the skin.

Examples of the pearl agent include white pigments such as titanium oxide, zinc oxide, cerium oxide, and barium sulfate; white extender powders such as talc, muscovite, phlogopite, lepidolite, biotite, synthetic mica, sericite, synthetic sericite, kaolin, silicon carbide, bentonite, smectite, silicic anhydride, aluminum oxide, magnesium oxide, zirconium oxide, antimony oxide, diatomaceous earth, aluminum silicate, aluminum magnesium metasilicate, calcium silicate, barium silicate, magnesium silicate, calcium carbonate, magnesium carbonate, hydroxyapatite, and boron nitride; glitter powders such as calcium aluminum borosilicate, titanium dioxide-coated mica, titanium dioxide-coated glass powders, titanium dioxide-coated bismuth oxychloride, titanium dioxide-coated mica, titanium dioxide-coated talc, iron oxide-coated mica, iron oxide-coated mica titanium, iron oxide-coated glass powders, Prussian blue-treated mica titanium, carmine-treated mica titanium, bismuth oxychloride, fish scale flake, polyethylene terephthalate-aluminum-epoxy laminated powders, and polyethylene terephthalate-polyolefin laminated film powders; organic low-molecular-weight powders such as N-acyllysine; metal powders such as an aluminum powder, a gold powder, and a silver powder; composite powders such as fine-particle titanium oxide-coated mica titanium, fine-particle zinc oxide-coated mica titanium, barium sulfate-coated mica titanium, titanium oxide-containing silicon dioxide, and zinc oxide-containing silicon dioxide; and non-crosslinked acrylic particles.

Examples of the soft focus agent include kapok fibers; polymethyl methacrylate crosspolymers; polymethyl methacrylate; (acrylates/ethylhexyl acrylate) crosspolymers; and natural organic powders such as a silk powder and a cellulose powder.

Examples of the lame agent include glitter powders such as calcium aluminum borosilicate, titanium dioxide-coated mica, titanium dioxide-coated glass powders, titanium dioxide-coated bismuth oxychloride, titanium dioxide-coated mica, titanium dioxide-coated talc, iron oxide-coated mica, iron oxide-coated mica titanium, iron oxide-coated glass powders, Prussian blue-treated mica titanium, carmine-treated mica titanium, bismuth oxychloride, fish scale flake, polyethylene terephthalate-aluminum-epoxy laminated powders, and polyethylene terephthalate-polyolefin laminated film powders. The lame agent may be, for example, powders produced by laminating about 100 layers composed of a plurality of resins having different refractive indices, for example, polymethyl methacrylate thin films and polyethylene terephthalate thin films, to provide pearly gloss due to the interference color.

The light-scattering layer 522 may contain only one reflective material or two or more reflective materials. Of these, titanium oxide, zinc oxide, cerium oxide, aluminum oxide, and magnesium oxide are preferred, and titanium oxide, zinc oxide, and cerium oxide are particularly preferred from the viewpoint that, for example, the color of the skin 510 (and the color of a complementary color layer 521 described below if the complementary color layer 521 is also formed) is unlikely to be visually recognized. The light-scattering layer 522 may optionally contains a fluorescent agent, Swarovski, beads, spangles, pearls, or the like.

The shape of the reflective material is not particularly limited and may be, for example, a spherical, plate-like, or acicular shape. However, the reflective material preferably has an average particle size of 125 nm or more and 2 µm or less. When the average particle size is 125 nm or more, for example, the color of the skin 510 is unlikely to be visually recognized. The average particle size is the median (D50) of the cumulative value of the particle size distribution measured by a laser diffraction method. Regarding the average particle size of the reflective material, the average particle size (D50) is more preferably 125 nm or more and 1,000 nm or less. Still more preferably, the average particle size (D50) is 125 nm or more and 1,000 nm or less, and a value (D90) at 90% of the cumulative value of the particle size distribution is 3,000 nm or less.

Examples of the binder contained in the light-scattering layer 522 include particles composed of (meth)acrylic resins such as alkyl (meth)acrylate polymers, styrene-(meth)acrylate copolymers, alkyl (meth)acrylate-vinyl acetate copolymers, (meth)acrylic acid-alkyl (meth)acrylate copolymers, and alkyl (meth)acrylate dimethicone polymers; vinyl acetate polymers; and vinylpyrrolidone-styrene copolymers. In the present specification, the term "(meth)acryl" refers to acryl, methacryl, or a mixture of acryl and methacryl.

The form of the binder is not particularly limited but is preferably a particle. The binder is particularly preferably formed of particles of a (meth)acrylic resin (hereinafter, also simply referred to as "acrylic particles"). If the binder is formed of acrylic particles, the reflective material tends to have good fixability, and the light-scattering layer 522 tends to have good durability. The binder is more preferably formed of particles of a (meth)acrylic resin that does not irritate the skin. Accordingly, the acrylic particles are preferably selected from, for example, the components listed in the list of cosmetic ingredient label names based on the Pharmaceutical Affairs Law in Japan, the components in accordance with EU cosmetics regulation (Cosmetics Directive 76/768/EEC), and the components described in International Cosmetic Ingredient Dictionary and Handbook (January 1, 2002, 9th edition) by the Cosmetic, Toiletry & Fragrance Association, U.S. (CTFA in the United States), and are preferably acrylic resin particles used in publicly known cosmetics and the like.

Specific examples of the (meth)acrylic resin constituting the acrylic particles include homopolymers of (meth)acrylic monomers, copolymers of two or more (meth)acrylic monomers, and copolymers of (meth)acrylic monomers and other monomers.

Examples of the (meth)acrylic monomers include acrylic acid, methyl acrylate, ethyl acrylate, acrylamide, n-propyl acrylate, n-butyl acrylate, isobutyl acrylate, octyl acrylate, 2-ethylhexyl acrylate, N,N-dimethylaminoethyl acrylate, acrylonitrile, methacrylic acid, ethyl methacrylate, methacrylamide, n-propyl methacrylate, n-butyl methacrylate, isobutyl methacrylate, 2-ethylhexyl methacrylate, octyl methacrylate, hydroxyethyl methacrylate, and N,N-dimethylaminoethyl methacrylate.

Examples of the other monomers that can be copolymerized with the (meth)acrylic monomers include styrene, vinyl acetate, silicone macromers, fluorinated monomers, and alkoxysilane unsaturated monomers.

In the case where the binder is particulate, the binder preferably has an average particle size of 30 nm to 150 nm. The average particle size is the median (D50) of the cumulative value of the particle size distribution measured by a laser diffraction method. More preferably, the average particle size (D50) is 30 nm to 150 nm, and a value (D90) at 90% of the cumulative value of the particle size distribution is 250 nm or less. When the binder has an average particle size in this range, the reflective material has high fixability.

When the amount of the reflective material is set to 10 parts by mass, the amount of binder contained in the light-scattering layer 522 is preferably 0.5 to 10 parts by mass, more preferably 1.5 to 5.7 parts by mass. When the amount of binder relative to the amount of reflective material is in the above range, the reflective material has high fixability. When the amount of binder is in this range, the amount of reflective material becomes relatively easily sufficient, and light can be sufficiently reflected or scattered by the light-scattering layer 522.

The film-forming agent, the dispersant, and various additives contained in the light-scattering layer 522 will be described in detail in the section of the cosmetic ink described below. Thus, a description thereof is omitted here.

The thickness of the light-scattering layer 522 is preferably 10 nm to 120 µm, more preferably 10 nm to 100 µm. When the light-scattering layer 522 has a thickness in the above range, light reflected at the surface of the skin 510 (and light reflected at the surface of the a complementary color layer 521 described below if the complementary color layer 521 is formed) is easily sufficiently reflected at the light-scattering layer 522.

### 3. Coloring Layer

The coloring layer 523 is a layer disposed on the light-scattering layer 522 and containing a colorant and may be, for example, a layer containing a colorant and a binder. The coloring layer 523 may also contain, for example, a film-forming agent, a dispersant, and various additives. By supermposing a skin color or a desired color on the light-scattering layer 522 by the coloring layer 523, a normal skin color or a desired color is observed from the surface of the cosmetic sheet 50.

Although the color of the coloring layer 523 may be usually adjusted to a color matching with the skin, the coloring layer 523 may be of any color when the cosmetic sheet 50 is used as cosmetics such as a cheek, eye shadow, or body painting. In the cosmetic sheet 50, the entirety of the coloring layer 523 may have the same color, that is, the color of the entire cosmetic sheet 50 may be uniform. Alternatively, the coloring layer 523 may partly have a different color region.

The coloring layer 523 may be formed of only one layer or two or more layes. When the coloring layer 523 is formed of a plurality of layers, the colorants contained in the layers may be of the same type or of different types. The amounts of colorants contained in the layers may be the same or different. For example, a coloring layer of any color may be stacked on a coloring layer of skin color

The color and brightness of the coloring layer 523 can be adjusted by, for example, a combination of colorants contained in the coloring layer 523. The color and brightness of the coloring layer are easily adjusted in desired ranges by, for example, further combining colorants of blue or black with colorants of white, red, and yellow.

Examples of the colorant contained in the coloring layer 523 include inorganic red pigments such as iron titanate, e.g., iron oxide and iron hydroxide; inorganic brownish pigments such as γ-iron oxide; inorganic yellowish pigments, such as yellow iron oxide and ocher; inorganic black pigments such as black iron oxide and carbon black; inorganic violet pigments such as manganese violet and cobalt violet; inorganic green pigments such as chromium hydroxide, chromium oxide, cobalt oxide, and cobalt titanate; inorganic bluish pigments such as Prussian blue (ferric ferrocyanide), ultramarine (ultramarine blue), lapis lazuli blue, mountain blue, aluminum-cobalt oxide, aluminum-zinc-cobalt oxide, silicon-cobalt oxide, silicon-zinc-cobalt oxide, cobalt pigments, smalt, cobalt blue, cobalt stannate, cobalt chromium blue, cobalt-aluminum-silicon oxide, and manganese blue; organic blue pigments and blue dyes such as indigo, phthalocyanine, indanthrene blue, and sulfonated products thereof; lakes produced from various tar-based dyes; lakes produced from various natural dyes; and composite synthetic resin powders produced by combining these powders.

The binder contained in the coloring layer 523 may be the same as the binder contained in the light-scattering layer 522 described above. The amount of binder contained in the coloring layer 523 is preferably 0.5 to 10 parts by mass, more preferably 1.5 to 5.7 parts by mass when the amount of the colorant is set to 10 parts by mass. When the amount of binder relative to the amount of colorant is in the above range, the colorant has high fixability. When the amount of binder is in the above range, the amount of colorant becomes relatively easily sufficient, and the coloring layer 523 of a desired color can be provided.

The thickness of the coloring layer 523 is appropriately selected according to, for example, the depth of a desired color and is preferably 10 nm to 15 µm, more preferably 10 nm to 3 µm. When the thickness of the coloring layer is in the above range, a desired color is easily developed without causing a feeling of thickness. When a plurality of coloring layers 523 are stacked, the total thickness of these layers is preferably in the above range.

### 4. Glossy Layer

The glossy layer 524 is a layer disposed on the coloring layer 523 and containing a gloss agent. For example, the glossy layer 524 may be a layer containing a gloss agent and a binder. The glossy layer 524 may also contain, for example, a film-forming agent, a dispersant, and various additives. In the glossy layer 524, a gloss agent 524 scatters or reflects light, and luster and transparency can be thereby provided to a region to which the cosmetic sheet 50 is attached. Preferably, the glossy layer 524 is disposed as the outermost surface layer of the cosmetic sheet 50, that is, disposed on a surface of the cosmetic sheet 50, the surface being opposite to the thin film 520.

The glossy layer 524 may be formed so as to cover the entire surface of the coloring layer 523 or formed so as to cover only a partial region of the coloring layer 523. If the glossy layer 524 is disposed only on a partial region, a portion on which the glossy layer 524 is disposed functions as a highlight or the like. As a result, it is possible to make the region to which the cosmetic sheet 50 is attached look three-dimensionally or make a part look brightly.

The glossy layer 524 may be formed of one layer or two or more layers. When the glossy layer 524 is formed of a plurality of layers, the gloss agents contained in the layers may be of the same type or of different types. The amounts of gloss agents contained in the layers may be the same or different.

The gloss agent contained in the glossy layer 524 may be formed of particles that scatter or reflect ultraviolet light and visible light (for example, light with a wavelength in the range of 200 nm to 780 nm). The gloss agent may be, for example, a white pigment, a pearl agent, a soft focus agent, a lame agent, or another luminescent/glossy material. Preferably, these gloss agents do not irritate the skin.

The pearl agent, the soft focus agent, and the lame agent may be the same as the pearl agent, the soft focus agent, and the lame agent, respectively, that are contained in the light-scattering layer 522. Examples of the other luminescent/glossy material include fluorescent agents, Swarovski, beads, spangles, and pearls.

The glossy layer 524 may contain only one of these gloss agents or two or more of the gloss agents. The shape of the gloss agent is not particularly limited and may be any shape such as a spherical, polygonal prism, or a scaly shape. Furthermore, the gloss agent preferably has an average particle size of 10 nm to 10 µm. The average particle size is the median (D50) of the cumulative value of the particle size distribution measured by a laser diffraction method. Regarding the average particle size of the gloss agent, the average particle size (D50) is more preferably 20 nm or more and 6 µm or less, and the average particle size (D50) is more preferably 20 nm or more and 4 µm or less. Still more preferably, a value (D90) at 90% of the cumulative value of the particle size distribution is 10 µm or less.

The binder contained in the glossy layer 524 may be the same as the binder contained in the light-scattering layer 522 described above. Herein, the binder in the glossy layer 524 and the binder in the coloring layer 523 preferably contain compounds having a common structure and particularly preferably contain the same compound. In the present specification, the term "compounds having a common structure" refers to compounds having a common skeleton or a common functional group. When the binder in the glossy layer 524 and the binder in the coloring layer 523 have a common structure, a glossy layer ink for forming the glossy layer 524 easily spreads sufficiently in the formation of the glossy layer 524 on the coloring layer 523. As a result, the glossy layer 524 can be uniformly formed in a desired region. In addition, these binders interact with each other to easily achieve very good adhesion between the glossy layer 524 and the coloring layer 523.

The amount of binder contained in the glossy layer 524 is preferably 0.5 to 10 parts by mass, more preferably 1.5 to 5.7 parts by mass when the amount of the gloss agent is set to 10 parts by mass. When the amount of binder relative to the amount of gloss agent is in the above range, the gloss agent has high fixability. When the amount of binder is in the above range, reflection and scattering of light by the gloss agent is unlikely to be hindered, and desired luster and transparency are easily obtained.

The thickness of the glossy layer 524 is appropriately selected according to a desired effect and is preferably 10 nm to 60 µm, more preferably 1 µm to 50 µm. When the thickness of the glossy layer 524 is in the above range, desired luster and transparency are easily obtained. When a plurality of glossy layers 524 are laminated, the total thickness of these layers is preferably in the above range.

### 5. Complementary Color Layer

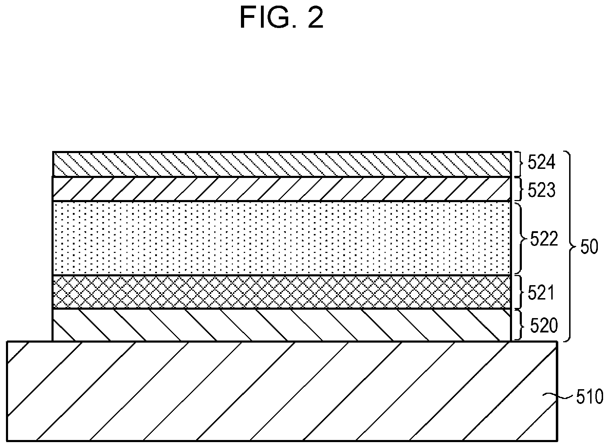

As described above, in the case where a cosmetic sheet 50 according to the present disclosure is attached to a discolored portion of a skin 510, as illustrated in a schematic sectional view of Fig. 2, a complementary color layer 521 may be disposed between a thin film 520 and a light-scattering layer 522. The complementary color layer 521 may be a layer containing a colorant complementary to the color of the discolored portion and a binder. The complementary color layer 521 may also contain, for example, a film-forming agent, a dispersant, and various additives.

In the present specification, colors that are complementary to each other are two colors, on a straight line passing through the white point in the chromaticity diagram, opposing to each other with the white point therebetween. In other words, two different colors that become achromatic (white, gray, or black) when mixed in an appropriate ratio are referred to as colors that are complementary to each other. When a color A in a spectrum is blocked, a color B obtained by collecting the remaining light is complementary to the color A. For example, if the color of the discolored region is red to orange, orange to yellow, or brown with low brightness, the complementary color layer may be a layer having a color tone of green to blue. In the present specification, the "color tone complementary" to the color of a discolored region includes not only the completely complementary color of the discolored region but also colors similar to the completely complementary color.

Examples of the discolored portion of the skin include pigmented spots, chloasmata, nevus spilus, melanocytic nevi, nevus of Ota, acquired dermal melanocytosis, erythema, purpura, leukoderma, bruise, moles, blackhead, sunburn regions, acne (pimples), pimple scars, pigmentation due to friction or inflammation, wrinkles, ephelides (freckles), tattoos, verrucae, and scars.

When the cosmetic sheet 50 includes the complementary color layer 521, light reflected in a discolored region of the skin 510 interferes with reflected light from the complementary color layer 521 to make reflected light from the discolored region achromatic. In general, if a layer of a color (for example, blue) complementary to the color of a discolored portion is formed, the color is easily visually recognized from the surface, and it is difficult to achieve a desired color of the cosmetic sheet. However, by disposing the complementary color layer 521 between the thin film 520 and the light-scattering layer 522, light reflected at the surface of the complementary color layer 521 is scattered or reflected by the light-scattering layer 522. Accordingly, in the cosmetic sheet 50 according to the present disclosure, even if the complementary color layer 521 is a layer of any color, the color is unlikely to be visually recognized from the surface of the cosmetic sheet 50, and the cosmetic sheet 50 can have a hue and a chromaticity in desired ranges.

Here, the complementary color layer 521 may be formed of one layer or two or more layers. When the complementary color layer 521 is formed of a plurality of layers, the colorants contained in the layers may be of the same type or of different types. The amounts of colorants contained in the layers may be the same or different.

The colorant contained in the complementary color layer 521 may be the same as the colorant contained in the coloring layer 523. The complementary color layer 521 may contain only one colorant or two or more colorants. The shape of the colorants is not particularly limited and may be any shape such as an acicular, irregular, spherical, or plate-like shape.

The median (D50) of the cumulative value of the particle size distribution of the colorant measured by a laser diffraction method is preferably 125 nm or more and 2 µm or less, more preferably 125 nm to 1,000 nm. Still more preferably, the average particle size (D50) is 125 nm to 1,000 nm, and a value (D90) at 90% of the cumulative value of the particle size distribution is 3,000 nm or less. When the average particle size (D50) of the particle size distribution of the colorant is in the above range, the complementary color layer 521 can have a thickness in a desired range, and the color of the complementary color layer 521 is unlikely to be visually recognized, as described above.

The binder contained in the complementary color layer 521 may be the same as the binder contained in the light-scattering layer 522. The amount of binder contained in the complementary color layer 521 is preferably 0.5 to 10 parts by mass, more preferably 1.5 to 5.7 parts by mass when the amount of colorant is set to 10 parts by mass. When the amount of binder relative to the amount of colorant is in the above range, the colorant has high fixability. In addition, when the amount of binder is in the above range, the amount of colorant becomes relatively easily sufficient, and the complementary color layer 521 can have a desired color.

Here, the thickness of the complementary color layer 521 is preferably 0.1 to 6 µm, more preferably 0.1 to 1 µm. The complementary color layer 521 having a thickness in the above range easily makes the color of a discolored region achromatic when the cosmetic sheet 50 is attached to the discolored region of the skin 510. When the complementary color layer 521 is formed of a plurality of layers, the total thickness of these layers is preferably in the above range.

### 6. Other Configurations

The cosmetic sheet 50 may include a layer other than the thin film 520, the light-scattering layer 522, the coloring layer 523, the glossy layer 524, and the complementary color layer 521 within a range that does not impair the objects and advantages of the present disclosure. For example, a hygroscopic layer that contains a moisture absorbent to control the humidity on the surface of the cosmetic sheet 50 and to enhance the comfort may be disposed between the coloring layer 523 and the light-scattering layer 524. Examples of the moisture absorbent include spherical silica, porous acrylic particles, and nylon 6 (polyamide 6).

A description has been made of an embodiment in which the thin film 520, the light-scattering layer 522, the coloring layer 523, and the glossy layer 524 are laminated in this order or an embodiment in which the thin film 520, the complementary color layer 521, the light-scattering layer 522, the coloring layer 523, and the glossy layer 524 are laminated in this order. Alternatively, for example, the glossy layer 524 may be disposed on a structure in which light-scattering layers 522 and coloring layers 523 are repeatedly laminated. Alternatively, the coloring layer 523 and the glossy layer 524 may be disposed on a structure in which complementary color layers 521 and light-scattering layers 522 are repeatedly laminated. In any configuration, the glossy layer 524 described above is preferably disposed as the outermost surface layer. With this configuration, luster and transparency can be imparted to a portion to which the cosmetic sheet 50 is attached.

### 7. Method for Producing Cosmetic Sheet

A method for producing the cosmetic sheet described above will be described below. The cosmetic sheet can be produced by performing, for example, a step of applying a light-scattering layer ink containing a reflective material to a surface of a thin film (the thin film described above), the surface being opposite to a surface to be attached to a skin, to form a light-scattering layer; a step of applying a coloring layer ink containing a colorant onto the light-scattering layer to form a coloring layer; and a step of applying a glossy layer ink containing a gloss agent to form a glossy layer. In the case where a complementary color layer is formed, before the formation of the light-scattering layer, a step of applying a complementary color layer ink containing a colorant to form a complementary color layer is performed.

Here, the light-scattering layer ink applied in the step of forming a light-scattering layer may be any ink containing a reflective material, for example, an ink containing the reflective material described above, the binder described above, a higher alcohol, and purified water. The ink may optionally contain, for example, a film-forming agent, a dispersant, and various additives.

The coloring layer ink applied in the step of forming a coloring layer may be any ink containing a colorant, for example, an ink containing the colorant described above, the binder described above, a higher alcohol, and purified water. The ink may optionally contain, for example, a film-forming agent, a dispersant, and various additives.

Furthermore, the glossy layer ink applied in the step of forming a glossy layer may be any ink containing a gloss agent, for example, an ink containing the gloss agent described above, the binder described above, a higher alcohol, and purified water. The ink may optionally contain a film-forming agent, a dispersant, and various additives.

The complementary color layer ink applied in the step of forming a complementary color layer may be any ink containing a colorant, for example, an ink containing the colorant described above, the binder described above, a higher alcohol, and purified water. The ink may optionally contain a film-forming agent, a dispersant, and various additives.

The light-scattering layer ink, the coloring layer ink, the glossy layer ink, and the complementary color layer ink may be prepared as inks that are different from each other and may be individually applied in each step. Alternatively, the light-scattering layer ink, the coloring layer ink, the glossy layer ink, and the complementary color layer ink may be prepared as common inks and may be applied in each step. Specifically, a white ink, a red ink, a yellow ink, a blue ink, a black ink, and the like may be prepared, and these inks may be appropriately combined to form desired layers. In some types of the cosmetic sheet, a white ink can be used as the light-scattering layer ink and the glossy layer ink.

Herein, in the production of a cosmetic sheet, the method for applying the light-scattering layer ink, the coloring layer ink, the glossy layer ink, and the complementary color layer ink (hereinafter, also collectively referred to as a cosmetic ink) is not particularly limited, and a publicly known method may be employed. Examples of the method for applying the complementary color layer ink, the light-scattering layer ink, and the coloring layer ink include an ink jet printing method, a screen printing method, offset printing, and gravure printing. Of these, the ink jet method is preferred from the viewpoint of, for example, facilitating on-demand printing and achieving multilayer printing in which the cosmetic ink is applied a plurality of times.

For example, an ink jet apparatus used when the cosmetic ink is applied by the ink jet method is not particularly limited. Any apparatus using a publicly known piezoelectric system, thermal system, or electrostatic system can be used. Of these, an ink jet apparatus using a piezoelectric element system is preferred from the viewpoint that heating, which is necessary for a thermal ink jet system, is not necessary.

The cosmetic ink may be applied only once or twice or more in each step of forming each layer. When the cosmetic ink is applied multiple times in each step, the cosmetic ink may be dried each time of application or may be dried after the cosmetic ink is applied multiple times.

The glossy layer ink may contain particles having a relatively large particle size. Accordingly, the method for applying the glossy layer ink is appropriately selected according to, for example, the particle size of particles contained in the glossy layer ink and an application pattern of the glossy layer ink. Examples of the method for forming the glossy layer ink include not only an ink jet printing method, a screen printing method, offset printing, and gravure printing but also spray printing, letterpress printing, flexographic printing, xerography, transfer, thermal transfer, silkscreen printing, and reverse roll coating using a gravure plate.

The light-scattering layer ink, the coloring layer ink, the glossy layer ink, and the complementary color layer ink may be applied from separated apparatuses or the same apparatus. For example, in the case where the light-scattering layer ink, the coloring layer ink, and the complementary color layer ink are applied by an ink jet printing method, and the glossy layer ink is applied by a spray printing method, each cosmetic ink may be applied by using an apparatus that includes an ink tank for containing each cosmetic ink, an ink jet nozzle connected to the ink tank, and a spray nozzle connected to the ink tank. The application of the cosmetic ink with the ink jet nozzle and the application of the cosmetic ink with the spray nozzle may be performed in a state where the thin film is fixed at a predetermined position. Alternatively, the cosmetic ink may be sequentially applied from the ink jet nozzle and the spray nozzle while the thin film is moved in a certain direction. An example of such an apparatus includes a transport unit for transporting a thin film, an ink jet printing unit for applying a cosmetic ink to the thin film transported by the transport means, and a spray printing unit for further applying a glossy layer ink onto a coating film (such as a complementary color layer, a light-scattering layer, or a coloring layer) formed by an ink jet device, the spray printing unit being disposed on the downstream side of the ink jet printing unit in a direction in which the thin film is transported. The apparatus may include, for example, a heating unit for drying coating films formed by the ink jet printing unit and the spray printing unit.

The method for drying the cosmetic ink may be any method that can remove the solvent (for example, a higher alcohol and purified water described below) in the cosmetic ink. For example, the cosmetic ink may be dried by a method including drying at atmospheric pressure and at room temperature or a method including drying by heating to a predetermined temperature and/or by reducing the pressure. In the case of heating, heating to a temperature of, for example, 25°C to 50°C is preferred. In the above range, the cosmetic ink can be efficiently dried without degrading the thin film and the solid component in the cosmetic ink. In the case of reducing the pressure, the pressure is preferably reduced by -0.1 to 0 MPa. Under the reduced pressure in the above range, the cosmetic ink can be efficiently dried. The drying time is appropriately selected according to, for example, the type of the cosmetic ink.

The timing to dry the cosmetic ink is not particularly limited. For example, after the application of all cosmetic inks (the complementary color layer ink, the light-scattering layer ink, the coloring layer ink, and the glossy layer ink) is finished, the cosmetic inks may be dried by the method described above. Alternatively, drying may be performed each time of application of a cosmetic ink.

### •Method for Preparing Cosmetic Ink

Each of the cosmetic inks is produced by, for example, mixing a colorant, a reflective material, a gloss agent, a binder, a higher alcohol, purified water, and other optional components in a disperser. The components can be mixed in a disperser such as a publicly known ball mill, sand mill, rolling mill, homomixer, or attritor. Hereafter, a description will be made of a higher alcohol, purified water, a film-forming agent, a dispersant, and various additives that are contained in the cosmetic inks in common.

### (Higher Alcohol)

The higher alcohol may be any higher alcohol that has 3 or more carbon atoms and that is compatible with purified water. The higher alcohol functions as a solvent for the cosmetic ink. After the application of the cosmetic ink, the higher alcohol is absorbed into the thin film or volatilizes.

The higher alcohol preferably has 3 to 5, more preferably 3 or 4 carbon atoms. The higher alcohol having such a number of carbon atoms is easily compatible with purified water.

The higher alcohol preferably contains a trivalent alcohol. When the higher alcohol contains a trivalent alcohol, the higher alcohol and purified water are unlikely to excessively volatilize inside various printers. Consequently, the cosmetic ink can be stably printed from the printers. In this case, since the viscosity of the cosmetic ink is maintained to be constant, desired images can be stably formed.

The trivalent alcohol may be any trivalent alcohol that does not irritate the skin and is preferably glycerin. Glycerin has high biological safety. In addition, when the cosmetic ink contains glycerin, aggregation of a colorant, a reflective material, a gloss agent, or the like is easily suppressed, and an increase in viscosity of the cosmetic ink is unlikely to occur even if the ink is stored for a long time.

The higher alcohol may contain a divalent alcohol or a monovalent alcohol. Examples of the divalent alcohol include diethylene glycol, propylene glycol, 1,3-propanediol, butylene glycol, and hexanediol. Examples of the monovalent alcohol include propanol, isopropanol, and butyl alcohol. Of these, a divalent alcohol is preferred, and propylene glycol is particularly preferred. Divalent alcohols have lower viscosity than purified water and trivalent alcohols and further have low surface tension. Accordingly, a divalent alcohol contained in the cosmetic ink enhances wettability of the cosmetic ink to the thin film or the like, and thus unevenness of the resulting image is unlikely to be generated.

The total amount of higher alcohol contained in 100 parts by mass of the cosmetic ink is preferably 20 parts by mass or less, more preferably 10 to 20 parts by mass. When the amount of higher alcohol is excessively large, a colorant, a reflective material, a gloss agent, or the like tends to aggregate. In contrast, when the amount of higher alcohol is 20 parts by mass or less, for example, aggregation of a reflective material, a colorant, a gloss agent, or the like is unlikely to occur, and the cosmetic ink can be stably ejected from various printers.

The amount of trivalent alcohol relative to 100 parts by mass of the cosmetic ink is appropriately selected according to the method for printing the cosmetic ink. For example, in the case where the cosmetic ink is printed from an ink jet apparatus, the amount is preferably 20 parts by mass or less, more preferably 10 to 20 parts by mass. When the amount of trivalent alcohol is in the above range, a higher alcohol and purified water in the cosmetic ink have moderately adjusted volatility, and the cosmetic ink can be stably ejected from an ink jet apparatus.

Furthermore, the amount of divalent alcohol relative to 100 parts by mass of the cosmetic ink is also appropriately selected according to the method for printing the cosmetic ink. For example, in the case where the cosmetic ink is printed from an ink jet apparatus, the amount is preferably 20 parts by mass or less, more preferably 10 to 20 parts by mass. When the amount of divalent alcohol is in the above range, the viscosity of the cosmetic ink easily falls within a desired range.

### (Purified Water)

Purified water also functions as a solvent of the cosmetic ink. After the printing of the cosmetic ink, purified water is absorbed into the thin film or volatilizes.

The purified water may be any purified water that is generally used for cosmetics and may be water purified by various methods such as distillation or ion exchange. For example, the purified water may be hot spring water, deep water, or water obtained by steam distillation of plants.

The amount of purified water relative to 100 parts by mass of the cosmetic ink is preferably 10 parts by mass or more, more preferably 20 parts by mass or more.

### (Film-Forming Agent)

The film-forming agent is a compound for enhancing film formability (for example, drying properties) of the cosmetic ink. In the present specification, the "film-forming agent" refers to a compound that can be dispersed in water at room temperature (excluding the component corresponding to the binder described above). The cosmetic ink may contain only one film-forming agent or two or more film-forming agents.

The film-forming agent may be a compound that can be dispersed or dissolved in a higher alcohol and/or purified water, for example, at least one compound selected from the group consisting of acrylic polymers, polysaccharide polymers, sugar alcohols, sterols, esters, and modified cornstarches. The cosmetic ink may contain only one film-forming agent or two or more film-forming agents. When the film-forming agent is a compound selected from the above group, the coating film formed from the cosmetic ink dries very rapidly.

Here, the film-forming agent preferably has an HLB value of 8 or more, more preferably 8 to19. When the film-forming agent has an HLB value of 8 or more, the film-forming agent is easily uniformly dispersed or dissolved in a higher alcohol, purified water, or the like. The HLB value is an index representing a relative affinity ratio for two liquids in an oil-water system. In general, a substance having a large HLB value has a high affinity for water. In the present specification, the HLB value is a value calculated by the Griffin method.

The film-forming agent is also preferably a material that does not irritate the skin. Examples of the acrylic polymers include an alkyl acrylate copolymer, a 2-amino-2-methyl-1-propanol salt (hereinafter, also referred to as "AMP") of an alkyl acrylate copolymer, a sodium salt (hereinafter, also referred to as "Na") of an alkyl acrylate copolymer, an alkyl acrylate copolymer ammonium, an acrylic acid-alkyl acrylate copolymer, an alkyl acrylate-diacetone acrylamide copolymer, an AMP-alkyl acrylate-diacetone acrylamide copolymer, a 2-amino-2-methyl-1,3-propanediol salt (hereinafter, also referred to as "AMPD") of an alkyl acrylate-diacetone acrylamide copolymer, a hydroxyethyl acrylate-methoxyethyl acrylate copolymer, a hydroxyethyl acrylate-butyl acrylate-methoxyethyl acrylate copolymer, an AMP-acrylates-C1-18 alkyl acrylate-C1-8 alkyl acrylamide copolymer, an alkyl acrylate-octylacrylamide copolymer, an acrylates-t-butylacrylamide copolymer, an acrylates-ethylhexyl acrylate copolymer, an acrylates copolymer, an AMP-acrylates copolymer, a Na acrylates copolymer, a polyurethane-14-AMP-acrylates copolymer, a vinyl acetate-butyl maleate-isobornyl acrylate copolymer, a styrene-alkyl acrylate copolymer, a styrene-acrylates copolymer, a styrene-acrylamide copolymer, polyurethane-1 (a compound represented by INCI name: POLYURETHANE-1), polyacrylate-22 (a compound represented by INCI name: POLYACRYLATE-22), tricontanyl polyvinylpyrrolidone (PVP), an (eicosene/vinylpyrrolidone) copolymer, and a (vinylpyrrolidone/hexadecene) copolymer.

Examples of the polysaccharide polymers include gum arabic, glucan, succinoglycan, carrageenan, karaya gum, tragacanth gum, guar gum, locust bean gum, galactomannan gum, xanthan gum, starch, carob gum, quince seed (Cydonia oblonga), casein, dextrin, gelatin, sodium pectate, sodium alginate, methylcellulose, ethylcellulose, carboxymethylcellulose, hydroxyethylcellulose, hydroxypropylcellulose, crystalline cellulose, O-[2-hydroxy-3-(trimethylammonio)propyl]hydroxyethylcellulose chloride, O-[2-hydroxy-3-(trimethylammonio)propyl]guar gum chloride, O-[2-hydroxy-3-(trimethylammonio)propyl]locust bean gum chloride, starch hydroxypropyltrimonium chloride, glyceryl glucoside, glycosyl trehalose, Tremella Fuciformis polysaccharide, and dextrin isostearate.

Examples of the sugar alcohols include sorbitol, maltitol, and glucose. The sterols are compounds having a sterol skeleton. Examples of the sterols include phytosterols such as campesterol, campestanol, brassicasterol, 22-dehydrocampesterol, stigmasterol, stigmastanol, 22-dihydrospinasterol, 22-dehydrostigmastanol, 7-dehydrostigmasterol, sitosterol, tirucallol, euphol, fucosterol, isofucosterol, codisterol, clionasterol, poriferasterol, clerosterol, 22-dehydroclerosterol, fungisterol, chondrillasterol, avenasterol, vernosterol, and pollinastanol; zoosterols such as cholesterol, dihydrocholesterol, cholestanol, coprostanol, epicoprosterol, epicoprostanol, 22-dehydrocholesterol, desmosterol, 24-methylenecholesterol, lanosterol, 24,25-dihydrolanosterol, norlanosterol, hydrogenated products spinasterol, dihydroagnosterol, agnosterol, lophenol, and lathosterol; mycosterols such as dehydroergosterol, 22,23-dihydroergosterol, episterol, ascosterol, and fecosterol; and hydrogenated products thereof.

Examples of the esters include dipentaerythritol fatty acid esters such as dipentaerythrityl hexa(hydroxystearate/stearate/rosinate), dipentaerythrityl (hydroxystearate/stearate/rosinate), dipentaerythrityl hexahydroxystearate, dipentaerythrityl tetra(hydroxystearate/isostearate), and dipentaerythrityl (hydroxystearate/isostearate);hydrogenated castor oil fatty acid esters such as hydrogenated castor oil stearate, hydrogenated castor oil isostearate, and hydrogenated castor oil hydroxystearate; cholesterol fatty acid esters such as cholesteryl hydroxystearate; phytosterol fatty acid esters such as phytosteryl oleate and phytosteryl macadamiate; hydrogenated vegetable oils such as hydrogenated coconut oil and hydrogenated palm oil; (phytosteryl/isosteryl/cetyl/stearyl/behenyl) dimer dilinoleate; sucrose pentahydroxystearate; and di(octyldodecyl/phytosteryl/behenyl) lauroyl glutamate.

The modified cornstarches may be compounds produced by modifying cornstarch with any compound within a range that does not impair the objects and advantages of the present disclosure. Examples thereof include hydroxypropyl-modified starches produced by causing 3-(dodecenyl)dihydro 2,5-furandione to react with cornstarch.

Among the compounds mentioned above, from the viewpoint of improving drying properties of the cosmetic ink, an acrylates copolymer, an acrylates (ethylhexyl acrylate) copolymer, a polyurethane-14-AMP-acrylates copolymer, alkyl acrylate copolymer ammonium, bis(behenyl/isostearyl/phytosteryl) dimer dilinoleyl dimer dilinoleate-triglyceryl hydrogenated rosinate, xanthan gum crosspolymer-hydroxyethylcellulose, Tremella Fuciformis polysaccharide, a modified cornstarch, and dextrin isostearate are preferred, and an acrylates copolymer, an acrylates (ethylhexyl acrylate) copolymer, a polyurethane-14-AMP-acrylates copolymer, alkyl acrylate copolymer ammonium, bis(behenyl/isostearyl/phytosteryl) dimer dilinoleyl dimer dilinoleate-triglyceryl hydrogenated rosinate, xanthan gum crosspolymer-hydroxyethylcellulose, and Tremella Fuciformis polysaccharide are more preferred.

The amount of film-forming agent contained in 100 parts by mass of the cosmetic ink is preferably 20 parts by mass or less, more preferably 0.1 to 10 parts by mass, still more preferably 0.3 to 5 parts by mass. When the amount of film-forming agent is 0.1 parts by mass or more, drying properties of the cosmetic ink is enhanced as described above. On the other hand, an excessively large amount of film-forming agent may excessively increase the viscosity of the cosmetic ink. However, 20 parts by mass or less of the film forming agent can provide the cosmetic ink with a viscosity suitable for printing by various printing methods.

In preparation of the cosmetic ink, the film-forming agent is typically dissolved in a solvent to prepare a solution, and the solution is mixed with, for example, a colorant, a reflective material, a gloss agent, a binder, a higher alcohol, and purified water. The solvent used in this case is also preferably a solvent that does not irritate the skin and is preferably the above higher alcohol and water.

The cosmetic ink may further contain, for example, a water-soluble polymer (polymer that does not correspond to the above film-forming agent) for binding the reflective material, the colorant, the gloss agent, and the like to a printing object within a range that does not impair the objects and advantages of the present disclosure.

### (Dispersant)

The dispersant is a component added in order to uniformly disperse components in the cosmetic ink and to maintain the dispersion state for long time. The type of dispersant is not particularly limited as long as the dispersant can exhibit such a function. In the cosmetic ink, in particular, a colorant, a reflective material, a gloss agent (hereinafter, also collectively referred to as "various particles"), and the like tend to settle with time. In view of this, the dispersant is preferably a material capable of suppressing settling of these.

Specifically, the dispersant is preferably a compound that adsorbs around various particles and that is capable of suppressing settling of various particles as a result of electrostatic repulsion or repulsion due to steric hindrance between dispersant particles. The various particles may be dispersed in a dispersion liquid adjusted to be basic in order to enhance dispersibility to produced water or a higher alcohol. The cosmetic ink containing various particles that have been subjected to such a treatment preferably contains anionic dispersant, and particularly preferably contains a dispersant formed of an organic polymer from the viewpoint of easily obtaining repulsive force due to steric hindrance or the like. The organic polymer preferably has 8 or more carbon atoms, more preferably 10 or more carbon atoms.

Specific examples of the dispersant formed of an anionic organic polymer include polyoxyethylene stearyl ether phosphates such as steareth-2 phosphate and steareth-3 phosphate; polyoxyethylene lauryl ether phosphates such as oleth-4 phosphate; polyoxyethylene alkyl (12-15) ether phosphates (the values in the parentheses represent the numbers of carbon atoms); sucrose fatty acid ester; and sodium laureth-11 carboxylate.

The amount of dispersant is appropriately selected according to the type of the dispersant. The dispersant is preferably contained in an amount of 2% to 50% by mass, more preferably 2% to 10% by mass relative to the cosmetic ink. When the amount of dispersant is in the above range, for example, settling of various particles is suppressed, and phase separation, settling, and the like can be made difficult to occur for a long time.

### (Various Additives)

The various additives are also preferably compounds having negative skin irritation. Examples of the various additives include pH-adjusting agents, thickeners, ultraviolet absorbers, ultraviolet scattering agents, antiseptic/antifungal agents, deoxidizers, antioxidants, antiseptic agents, antifading agents, antifoaming agents, fragrances, and solvents other than higher alcohols and purified water.

Examples of the pH-adjusting agents include acids such as hydrochloric acid, citric acid, acetic acid, phosphoric acid, sulfuric acid, gluconic acid, and succinic acid; carbonates such as potassium carbonate and sodium hydrogencarbonate; hydroxides of an alkali metal such as sodium hydroxide and potassium hydroxide; and buffer solutions containing any of these salts (such as a phosphate buffer solution, a citrate buffer solution, and an acetate buffer solution).

### • Physical Properties of Cosmetic Ink

Each of the cosmetic inks preferably has a viscosity of 50 mPa·s or less, more preferably 1 to 20 mPa·s, still more preferably 3.5 to 8 mPa·s, at 25°C, as measured with a cone-plate viscometer at a rate of 100 (1/s). The cosmetic ink having a viscosity in the above range can be easily printed by using various printers. In particular, the cosmetic ink having a viscosity in the above range is easily and stably ejected from an ink jet apparatus.

The cosmetic ink preferably has a pH in the range of 6 to 10, more preferably 7.5 to 9.5. When the cosmetic ink has a pH in the above range, for example, the cosmetic ink does not erode members of various printers. Furthermore, even if such a cosmetic ink is stored for a long time, for example, aggregation of a colorant, a reflective material, and a gloss agent is unlikely to occur, and a desired coating film is easily formed.

The cosmetic ink preferably has a surface tension of 50 mN/m or less, more preferably 32 mN/m to 46 mN/m, at 25°C. When the cosmetic ink having a surface tension of 50 mN/m or less is applied from various printers to various printing objects, the cosmetic ink has good wettability, and a film with a uniform thickness can be formed. The surface tension can be measured by various measurement methods. The above values are values measured by the pendant-drop method, which is employed in general-purpose devices.

Furthermore, the average particle size of particles contained in the cosmetic ink (except the glossy layer ink), that is, the median (D50) of the cumulative value of the particle size distribution measured by a laser diffraction method is preferably 125 nm or more and 2 µm. The average particle size (D50) is more preferably 125 nm or more and 1,000 nm or less. Still more preferably, the average particle size (D50) is 125 nm or more and 1,000 nm or less, and a value (D90) at 90% of the cumulative value of the particle size distribution is 3,000 nm or less. When the particles contained in the cosmetic ink have an average particle size in the above range, the cosmetic ink can be stably ejected from various printers, in particular, an ink jet apparatus. When the values of D50 and D90 are in the above ranges, the cosmetic ink can be stably ejected from an ink jet apparatus or the like.

In addition, the cosmetic ink preferably has negative skin irritation, that is, preferably has high biological safety. The cosmetic ink having negative skin irritation can also be used in applications involving contact with the skin or the like. In the present disclosure, the term "negative skin irritation" means that cell viability is more than 50% when a test is conducted with a three-dimensional skin model which is an alternative method to the skin irritation test. In the alternative method to the skin irritation test, a 5% sodium dodecyl sulfate (SDS) solution is used as an irritation control, and a phosphate-buffered saline (PBS) is used as a negative control. After the ink is exposed to the three-dimensional skin model for 18 hours, the cell viability is evaluated by MTT assay.

In an example of a method of making skin irritation of the cosmetic ink negative, all the components contained in the cosmetic ink are selected from the components listed in the list of cosmetic ingredient label names based on the Pharmaceutical Affairs Law in Japan, the components in accordance with EU cosmetics regulation (Cosmetics Directive 76/768/EEC), and the components described in International Cosmetic Ingredient Dictionary and Handbook (January 1, 2002, 9th edition) by the Cosmetic, Toiletry & Fragrance Association, U.S. (CTFA in the United States).

### • Regarding Production of Cosmetic Sheet Using Makeup Support System

As described above, the cosmetic sheet may be produced according to the skin of a particular individual. Such a cosmetic sheet according to a particular individual can be produced by a publicly known makeup support system. The publicly known makeup support system may be, for example, a system including an image-processing apparatus that includes an illumination unit, a camera, and a display unit such as a liquid crystal display with a touch screen; and a printer connected to the image-processing apparatus so as to communicate with the image-processing apparatus.

In the makeup support system, for example, the skin (for example, the face) of a user is photographed with the camera while the image-processing apparatus applies visible light from the illumination unit. The color of the coloring layer, a formation pattern of the glossy layer, and the like are determined according to the color and the condition of the skin and the request from the user to generate print data. In this case, the image-processing apparatus generates print data according to the individual size or the arrangement on the basis of the feature point information of the individual analyzed by an image analyzer. The image-processing apparatus may determine not only the color of the coloring layer but also, for example, a region in which the glossy layer is formed (portion to be highlighted) on the basis of the skin information and skeleton information of the individual analyzed by the image analyzer and generate the print data. The mage-processing apparatus then transmits the print data to the printer connected via a predetermined network or a cable. Thus, a cosmetic sheet is produced.

### EXAMPLES

Hereafter, the present disclosure will be described with reference to Examples. Examples do not limit the scope of the present disclosure.

### 1. Preparation of Cosmetic Ink

### 1-1. Materials

Materials used in Examples are as follows. The average particle size of particles is the median (D50) of the cumulative value of a particle size distribution measured by a laser diffraction method.
(A) Colorant
   Reddish coloriant: inorganic reddish pigment (average particle size: 150 nm)
   Yellowish colorant: inorganic yellowish pigment (average particle size: 150 nm)
   Bluish colorant: inorganic bluish pigment (average particle size: 150 nm)
   Blackish colorant: inorganic blackish pigment (average particle size: 150 nm)
   Whitish colorant: inorganic whitish pigment (reflective material) (average particle size: 950 nm)
   Whitish colorant: inorganic whitish pigment (gloss agent) (average particle size: 3.4 µm)
(B) Higher alcohol
   Glycerin
   1,3-Propanediol
(C) Purified water
(D) Binder
   Acrylic polymer particles (average particle size: 50 nm)
(E) Dispersant
   Polyoxyethylene stearyl ether phosphate
   Polyoxyethylene lauryl ether phosphate
   Polyoxyethylene alkyl (12-15) ether phosphate (the values in the parentheses represent the numbers of carbon atoms)
   Sodium metaphosphate
   Sodium pyrophosphate
   Sucrose fatty acid ester

### 1-2. Preparation of Light-Scattering Layer Ink and Coloring Layer Ink

The materials were mixed in the component ratio shown in Table 1 below to prepare a light-scattering layer ink and coloring layer inks. The light-scattering layer ink and a white ink for the coloring layer inks were used in common. Table 1 also shows the physical properties of the light-scattering layer ink and the coloring layer inks.

**[Table 1]**

| | | White ink | Red ink | Yellow ink | Blue ink | Black ink |
|---|---|---|---|---|---|---|
| (A) Colorant | Reddish coloriant (mass%) | | 10 to 15 | | | |
| | Yellowish colorant (mass%) | | | 10 to 15 | | |
| | Bluish colorant (mass%) | | | | 10 to 15 | |
| | Blackish colorant (mass%) | | | | | 10 to 15 |
| | Whitish colorant (mass%) | 10 to 15 | | | | |
| (B) Higher alcohol | Glycerin (mass%)/1,3-Propanediol (mass%) | 10/10 | | | | |
| (C) Purified water (mass%) | | 62.5 to 67.5 | | | | |
| (D) Binder | Acrylic polymer particles (mass%) | 2.5 | | | | |
| Viscosity (mPa·s) | | 5.5 | 5.5 | 5.5 | 5.5 | 5.5 |
| pH | | 8.0 | 8.0 | 8.0 | 8.0 | 8.0 |
| Surface tension (mN/m) | | 34 | 34 | 34 | 34 | 34 |

### 1-3. Preparation of Glossy Layer Ink

The materials were mixed in the component ratio shown in Table 2 below to prepare glossy layer inks. As shown in Table 3, the amount of each dispersant was changed in the range of 2 parts by mass to 10 parts by mass (the amount of (C) purified water was adjusted according to the amount of dispersant), and dispersibility of each of the glossy layer inks was evaluated. The dispersibility was evaluated as follows on the basis of a dispersion state that was visually observed when the ink was allowed to stand at 25°C for nine days after preparation. Table 3 shows the results.
A: Each component is uniformly dispersed.
B: Although a concentration gradient is present near the liquid surface, each component is dispersed.
C: The gloss agent obviously settles.

**[Table 2]**

| | | No. 1 | No. 2 | No. 3 | No. 4 | No. 5 | No. 6 | No. 7 |
|---|---|---|---|---|---|---|---|---|
| (A) Colorant | Inorganic whitish pigment (gloss agent) (mass%) | 10 to 15 | | | | | | |
| (B) Higher alcohol | Glycerin (mass%)/1,3-Propanediol (mass%) | 10/10 | | | | | | |
| (C) Purified water (parts bv mass) | | 52.5 to 67.5 | | | | | | |
| (D) Binder | Acrylic polymer particles (mass%) | 2.5 | | | | | | |
| (E) Dispersant | Polyoxyethylene stearyl ether phosphate (mass%) | 2 to 10 | - | - | - | - | - | - |
| | Polyoxyethylene lauryl ether phosphate (mass%) | - | 2 to 10 | - | - | - | - | - |
| | Polyoxyethylene alkyl (12-15) ether phosphate (mass%) | - | - | 2 to 10 | - | - | - | - |
| | Sodium metaphosphate (mass%) | - | - | - | 2 to 10 | - | - | - |
| | Sodium pyrophosphate (mass%) | - | - | - | - | 2 to 10 | - | - |
| | Sucrose fatty acid ester (mass%) | - | - | - | | | 2 to 10 | |

**[Table 3]**

| Type of dispersant | None | Anionic high-molecular-weight | Anionic high-molecular-weight | Anionic high-molecular-weight | Anionic low-molecular-weight | Anionic low-molecular-weight | Nonionic high-molecular-weight |
|---|---|---|---|---|---|---|---|
| Dispersant concentration | No. 7 | No. 1 | No. 2 | No. 3 | No. 4 | No. 5 | No. 6 |
| 0 (mass%) | C | - | - | - | - | - | - |
| 2 (mass%) | - | B | B | B | C | C | C |
| 4 (mass%) | - | B | B | B | C | C | C |
| 6 (mass%) | - | B | B | B | C | C | C |
| 8 (mass%) | - | B | A | B | C | C | C |
| 10 (mass%) | - | A | A | A | C | C | C |

As shown in Table 3, in the cases where anionic high-molecular-weight dispersants were used (No. 1 to No. 3), the dispersibility after the ink was allowed to stand for nine days was good. In addition, in the case of using these dispersants, the dispersibility enhanced with the increase in the amount of dispersant.

In contrast, in the cases where no dispersant was added (No. 7), anionic low-molecular-weight dispersants were used (No. 4 and No. 5), and a nonionic high-molecular-weight dispersant was used (No. 6), before the elapse of time from the preparation of the cosmetic ink, each component was uniformly dispersed, however, after the ink was allowed to stand for nine days, sedimentation of the gloss agent occurred regardless of the amount of dispersant added.

### 2. Production of Cosmetic Sheet

### 2-1. Example

The light-scattering layer ink and the coloring layer inks (the white ink, the red ink, the yellow ink, the blue ink, and the black ink) were charged into ink tanks of an ink jet apparatus including an LB3 ink jet head manufactured by Panasonic Precision Devices Co., Ltd. A printing object was prepared by bonding a polylactic acid sheet having a thickness of 200 nm to a support formed of filter paper.

The while ink was then printed on the printing object, and the resulting sheet after printing was dried at 40°C under reduced pressure (-0.5 kPa) for 60 minutes to form a light-scattering layer. Another identical light-scattering layer was further formed on the light-scattering layer so that the resulting light-scattering layer had a total thickness of 1,600 nm.

Subsequently, the inks of respective colors were printed and dried at 40°C under reduced pressure (-0.5 kPa) for 60 minutes to form a coloring layer having a color tone of skin color. The pattern of the coloring layer was a circular pattern concentric with the light-scattering layer. Two identical coloring layers were further formed on the coloring layer so that the resulting coloring layer had a total thickness of 1,600 nm.

The glossy layer ink (the ink of No. 2 (dispersant content: 8% by mass)) was applied onto the coloring layer by using a commercially available foundation ink spray (trade name: Hollywood Air). The sheet after printing was dried d at 40°C under reduced pressure (-0.5 kPa) for 60 minutes to form a glossy layer. The glossy layer had a thickness of 10 µm.

### 2-2. Comparative Example 1

A cosmetic sheet was produced as in the Example (the thicknesses of the layers are also the same) except that the glossy layer, the light-scattering layer, and the coloring layer were formed on the printing object in this order.

### 2-3. Comparative Example 2

The above gloss agent was mixed with each of the coloring layer inks (the white ink, the red ink, the yellow ink, the blue ink, and the black ink) and uniformly dispersed. The amounts of the gloss agent were each 10% to 15% by mass, and the amount of purified water was decreased according to the amount of gloss agent added.

A light-scattering layer was then formed on a printing object as in the Example. Subsequently, the coloring layer inks containing the gloss agent was printed on the light-scattering layer. The sheet after printing was dried at 40°C under reduced pressure (-0.5 kPa) for 60 minutes to form a coloring layer having a color tone of skin color.

### 3. Evaluation

The cosmetic sheets produced in Example and Comparative Examples 1 and 2 were disposed on a skin model, and luster and transparency were evaluated by visual observation. According to the results, in the cosmetic sheet of Example in which the glossy layer was formed as the outermost surface layer, the cosmetic sheet had luster (glossiness), and a sense of transparency was also felt. In addition, the degree of feeling of thickness was also low. When the cosmetic sheet was attached to the skin, the difference between a portion to which the cosmetic sheet was attached and a portion to which the cosmetic sheet was not attached was obscure. In contrast, the cosmetic sheet of Comparative Example 1 in which the glossy layer was disposed closer to the thin film than the coloring layer had a matte texture and did not have sufficient luster. Similarly, in the case where the gloss agent was added into the coloring layer, a matte texture was obtained. Accordingly, it was shown that sufficient luster and transparency were exhibited by disposing, on the coloring layer, a gloss agent in the form of a layer without mixing with another colorant or the like.

### Industrial Applicability

The cosmetic sheet according to the present disclosure does not cause a feeling of thickness and further exhibits luster and transparency when attached to the skin. Accordingly, the cosmetic sheet according to the present disclosure is useful as a sheet used the purpose of, for example, coloration and beautification of the skin.

### Reference Signs List

- 50: cosmetic sheet
- 510: skin
- 520: thin film
- 521: complementary color layer
- 522: light-scattering layer
- 523: coloring layer
- 524: glossy layer
- C1, C2, C3: light

## Claims

1. A cosmetic sheet to be attached to a skin, the cosmetic sheet comprising:
a thin film, one surface of which is to be attached to a skin;
a light-scattering layer disposed on the thin film and containing a reflective material;
a coloring layer disposed on the light-scattering layer and containing a colorant; and
a glossy layer disposed on the coloring layer and containing a gloss agent.

2. The cosmetic sheet according to Claim 1,
wherein the glossy layer is disposed so as to cover an entire surface of the coloring layer.

3. The cosmetic sheet according to Claim 1,
wherein the glossy layer is disposed so as to cover only a partial region of the coloring layer.

4. The cosmetic sheet according to any one of Claims 1 to 3,
wherein the glossy layer is disposed as an outermost surface layer.

5. The cosmetic sheet according to any one of Claims 1 to 4,
wherein the glossy layer has a thickness of 10 nm or more and 60 µm or less.

6. The cosmetic sheet according to any one of Claims 1 to 5, the cosmetic sheet being used for obscuring a discolored portion of a skin,
further comprising a complementary color layer disposed between the thin film and the light-scattering layer and containing a colorant complementary to a color of the discolored portion.

7. A method for producing a cosmetic sheet to be attached to a skin, the method comprising:
a step of applying a light-scattering layer ink containing a reflective material to a surface of a thin film, the surface being opposite to a surface to be attached to a skin, to form a light-scattering layer;
a step of applying a coloring layer ink containing a colorant onto the light-scattering layer to form a coloring layer; and
a step of applying a glossy layer ink containing a gloss agent onto the coloring layer to form a glossy layer.

8. The method for producing a cosmetic sheet according to Claim 7, wherein
the coloring layer ink and the glossy layer ink each contain a higher alcohol having 3 or more carbon atoms, purified water, and a binder, and
the binder in the glossy layer ink and the binder in the glossy layer ink contain compounds having a common structure.

9. The method for producing a cosmetic sheet according to Claim 7 or 8,
wherein the glossy layer ink further contains a film-forming agent.

10. The method for producing a cosmetic sheet according to Claim 9,
wherein an amount of the film-forming agent in the glossy layer ink is 0.1% to 10% by mass.

11. The method for producing a cosmetic sheet according to any one of Claims 7 to 10,
wherein the glossy layer ink further contains a dispersant.

12. The method for producing a cosmetic sheet according to any one of Claims 7 to 11,
wherein the application of the light-scattering layer ink and the applciation of the coloring layer ink are performed by an ink jet printing method.

13. The method for producing a cosmetic sheet according to Claim 12,
wherein the application of the glossy layer ink is performed by at least one method selected from a spray method, a screen printing method, an offset printing method, a gravure printing method, a letterpress printing method, a flexographic printing method, xerography, transfer, thermal transfer, a silkscreen printing method, and a reverse roll coating method using a gravure plate.

14. A cosmetic ink comprising:
a gloss agent, a higher alcohol having 3 or more carbon atoms, purified water, and a binder.

15. A printing ink comprising:
the cosmetic ink according to Claim 14.

16. An apparatus for producing a cosmetic sheet, comprising:
an ink tank for containing a cosmetic ink;
an ink jet nozzle connected to the ink tank; and
a spray nozzle connected to the ink tank.
